# EUROPÄISCHE PATENTSCHRIFT

(11) **EP 2 168 489 B1**
(45) Veröffentlichungstag und Bekanntmachung des Hinweises auf die Patenterteilung: **29.06.2011**
(21) Anmeldenummer: 09154833.9
(22) Anmeldetag: 11.03.2009
(51) Int. Cl.: A61B 6/03, A61B 6/00, A61B 6/04

(54) **Röntgengerät zur Brustuntersuchung im Stehen**
X-ray device for mammography in a standing postion
Dispositiv à rayons X pour mammographie d'une patiente debout

(30) Priorität: 29.09.2008 DE 102008042430
(43) Veröffentlichungstag der Anmeldung: 31.03.2010
(73) Patentinhaber: MIR Medical Imaging Research Holding GmbH, 91096 Möhrendorf (DE); Friedrich-Alexander-Universität Erlangen-Nürnberg, 91054 Erlangen (DE)
(72) Erfinder: Kalender, Willi, 91096, Möhrendorf (DE); Schilling, Harry, 85072, Eichstätt (DE)
(74) Vertreter: Lohr, Georg

(56) Entgegenhaltungen:
- EP-A- 1 864 611
- WO-A-94/06352
- WO-A-2008/054279
- DE-C1- 19 639 975
- US-A- 5 664 569
- US-A1- 2007 238 957
- US-A1- 2008 037 703
- US-B1- 6 463 122

## Beschreibung

### Technisches Gebiet

Die Erfindung betrifft ein Röntgengerät zur Abbildung der weiblichen Brust (Mammografie). Insbesondere betrifft die Erfindung ein Gerät, mit dem Reihenuntersuchungen effizient durchgeführt werden können.

### Stand der Technik

Zur Untersuchung der weiblichen Brust sind Röntgengeräte bekannt, bei denen die zu untersuchende Person auf einer Liege platziert wird. Ein solches Gerät ist beispielsweise in der US 6,480,565 offenbart. Eine Verkürzung der Untersuchungszeit ermöglichen Röntgengeräte, wie in der US 5,386,447, bei denen die zu untersuchende Person vor dem Röntgengerät steht. Aus der US 2007/0092059 A1 ist ein weiteres Röntgengerät bekannt, bei dem ein Strahler sowie ein Plattenförmiger Detektor in einem Halbkreis um die zu untersuchende Brust bewegt werden kann. Es könnten hier an verschiedenen Positionen aus verschiedenen Winkeln Aufnahmen angefertigt werden. Nachteilig an diesem Stand der Technik ist, dass auch hier einerseits eine durch den plattenförmigen Detektor beschränkte Auflösung realisierbar ist und andererseits eine relativ lange Aufnahmezeit benötigt wird, da zwischen den einzelnen Aufnahmen der Strahler und der Detektor neu positioniert werden müssen. Somit ist eine solche Anordnung nur bedingt für Reihenuntersuchungen geeignet.

In der US 2008/037703 A1, derEP-A-1864611, und der US-B1-6463122 sind Röntgengeräte zur Untersuchung der weiblichen Brust offenbart, in denen ein Röntgenstrahler zusammen mit einem Detektor um die Brust rotiert.

### Darstellung der Erfindung

Der Erfindung liegt die Aufgabe zugrunde, ein Röntgengerät zu gestalten, welches die weibliche Brust diagnostisch exakt, schnell und kostengünstig abbildet, und dabei die Strahlenbelastung weitestgehend auf die Brust beschränkt. Dabei sollen der Patientin möglichst geringe Schmerzen, durch Kompression der Brust etc. zugefügt werden und die Geräteressource optimal ausgenutzt werden.

Diese Aufgabe wird durch ein Röntgengerät nach Anspruch 1 gelöst. Vorteilhafte Ausgestaltungen der Erfindung sind in den Unteransprüchen angegeben.

Ein erfindungsgemäßes Röntgengerät zur Abbildung einer weiblichen Brust umfasst eine um eine Drehachse 12 drehbare Gantry 10. An dieser Gantry ist eine Röntgeneinrichtung mit Röntgenröhre 15 sowie einem Röntgendetektor 16 angebracht. Die Röntgenröhre 15 emittiert Röntgenstrahlung in Richtung des Röntgendetektors 16. Im Strahlengang zwischen der Röntgenröhre 15 und den Röntgendetektor 16 ist eine Fixiervorrichtung 40a, 40b angeordnet. Diese Fixiervorrichtung 40a, 40b dient zur Aufnahme beziehungsweise Fixierung einer weiblichen Brust 18. Zur Abbildung der Brust 18 rotiert die Gantry um die Drehachse. Gleichzeitig erfolgt durch die Vorschubeinrichtung 80 eine lineare Verschiebung zwischen der Gantry 10 und der in der Fixiervorrichtung 40a, 40b gehaltenen Brust 18. Wahlweise kann hier die in der Fixiervorrichtung 40a, 40b gehaltenen Brust 18 gegenüber der Gantry 10 verschoben werden. Besonders vorteilhaft ist es jedoch, wenn die Position der Brust nicht verändert wird und daher die Gantry 10 gegenüber der in der Fixiervorrichtung 40a, 40b gehaltenen Brust 18 verschoben wird. Die Richtung der Bewegung ist vorzugsweise parallel zur Drehachse 12 der Gantry. Die Bewegung kann wahlweise kontinuierlich mit konstanter Geschwindigkeit oder proportional zur Rotation der Gantry erfolgen. Alternativ kann die Bewegung auch in Schritten erfolgen, so dass beispielsweise nach jeder Umdrehung der Gantry ein Versatz um die Breite des Detektors erfolgt.

Durch den die Innenbohrung 17 der Gantry und durch den Bereich, in dem die Gantry 10 durch die Vorschubeinrichtung 80 verschiebbar ist, kann das Messfeld des Röntgengerätes definiert werden. Dieses Messfeld hat eine zylindrische Form. Der zu untersuchende Bereich der Brust muss in das Messfeld 18 hineinragen.

Durch die erfindungsgemäße Ausgestaltung des Röntgengerätes ist es nicht mehr notwendig, einen großen Flächendetektor einzusetzen, welcher die ganze Brust abbilden kann. Vielmehr genügt es einen kleineren Röntgendetektor 16 mit einer Breite in einer Richtung parallel zur Rotationsachse, welche wesentlich kürzer als die Länge der Brust 18 in der Fixiervorrichtung 40a, 40b ist. Damit kann in der Erfindung ein Röntgendetektor 16 mit einer wesentlich höheren Auflösung eingesetzt werden. Es können dreidimensionale Daten mit hoher diagnostischer Aussagekraft erzeugt werden. Eine vorteilhafte Aufnahmetechnik im Sinne der Erfindung ist die Spiral-CT-Technik. Hier wird das Messfeld durch eine Spiralbewegung abgetastet, dadurch kann eine vollständige Abbildung in wenigen Sekunden durchgeführt werden. Damit kann auch die pro zu behandelnder Person benötigte Zeit wesentlich reduziert werden.

Die Fixiervorrichtung 40a, 40b weist wenigstens eine Öffnung zur Aufnahme einer weiblichen Brust auf. Vorteilhaft ist eine Fixierung mithilfe einer Tasse bzw. Glocke in der die Brust durch Unterdruck gehalten wird. Aber auch andere Fixiermethoden mit Ringen, Schnüren, mechanischen oder adhäsiven Vorrichtungen sind möglich, die Fixierung ist derart gestaltet, dass die Hauptachse der Brust, gebildet durch die Senkrechte zur Brustwand und der Brustwarze, horizontal entlang des Rotationsachse der Gantry verläuft. Besonders bevorzugt ist es, wenn auf beiden Seiten der Gantry jeweils eine Fixiervorrichtung vorgesehen ist. Durch diese Anordnung kann bereits eine in einer Fixiervorrichtung positionierte Brust abgebildet werden, während eine Brust in einer zweiten Fixiervorrichtung positioniert wird. Damit ist ein nahezu kontinuierlicher Betrieb der Röntgeneinrichtung auf der Gantry möglich. So lassen sich effiziente Reihenuntersuchungen durchführen. Die Fixiervorrichtungen sind vorteilhafterweise austauschbar. So kann jede Fixiervorrichtung entsprechend der Größe der Brust der zu untersuchenden Person angepasst und/oder nach der Untersuchung ausgetauscht werden.

In einer weiteren Vorteilhaftausgestaltung der Erfindung ist das Röntgengerät hinter einer dünnen Wand angeordnet, die an ein Untersuchungszimmer angrenzt. Vom Untersuchungszimmer aus ist nur die Fixiervorrichtung sichtbar. Ebenso kann auf beiden Seiten des Röntgengerätes jeweils ein Untersuchungszimmer angeordnet sein, welches mit einer dünnen Wand gegenüber dem Röntgengerät abgeschlossen ist. Die Gantry kann nun mittels der Vorschubeinrichtung zwischen dem einen Teil der Fixiervorrichtung, welches einen ersten Untersuchungszimmer zugeordnet ist und einem zweiten Teil der Fixiervorrichtung, welches einen zweiten Untersuchungszimmer zugeordnet ist, bewegt werden.

Das erfindungsgemäße Röntgengerät kann wahlweise mit einem Standfuß zur Aufstellung auf einem Fußboden oder mit einer Wandhalterung zur Wandmontage ausgestattet sein.

In einer weiteren Ausgestaltung der Erfindung ist die Gantry 10 in der Höhe verstellbar, so dass er an verschiedene Körpergrößen der zu untersuchende Personen anpassbar ist. Um eine einfache Höhenverstellung zu ermöglichen ist vorteilhafterweise ein Gewichtsausgleich durch Federn oder Ausgleichsgewichte vorgesehen. Besonders bevorzugt ist es, wenn er die Höhenverstellung durch eine Hubvorrichtung, bevorzugt durch einen Motor, besonders bevorzugt durch einen Elektromotor erfolgt.

Weiterhin ist es vorteilhaft, wenn eine höhenverstellbare Trittstufe 27 beziehungsweise Standfläche vorgesehen ist, auf der die zu untersuchende Person steht. Auch diese Trittstufe kann durch eine Hubvorrichtung, bevorzugt durch einen Motor, besonders bevorzugt durch einen Elektromotor in der Höhe verstellt werden.

Besonders günstig ist dies, wenn die Fixierung der Brust 18 in der Fixiervorrichtung 40a, 40b mittels Unterdruck beziehungsweise Vakuum erfolgt. Dadurch wird eine unerwünschte Kompression der Brust vermieden.

Besonders bevorzugt ist das Röntgengerät ein Spirat-CT-Gerät.

Ebenso bevorzugt ist es, wenn er das Röntgengerät ein sequenzielles CT Gerät ist. Die Gantry eines solchen Gerätes bewegt sich zunächst entlang einer Kreisbahn und wird dann durch die Vorschubeinrichtung um einen bestimmten Weg verschoben.

### Beschreibung der Zeichnungen

Die Erfindung wird nachstehend ohne Beschränkung des allgemeinen Erfindungsgedankens anhand von Ausführungsbeispielen unter Bezugnahme auf die Zeichnungen exemplarisch beschrieben.
Figur 1 zeigt beispielhaft ein erfindungsgemäßes Röntgengerät mit einer zwischen zwei Untersuchungsräumen angeordneten Gantry.
Figur 2 zeigt eine Variante der Erfindung mit höhenverstellbarer Trittstufe.
Figur 3 zeigt eine Variante der Erfindung mit höhenverstellbarer Gantry.
Figur 4 zeigt eine Variante mit vorgesetzter Wand.

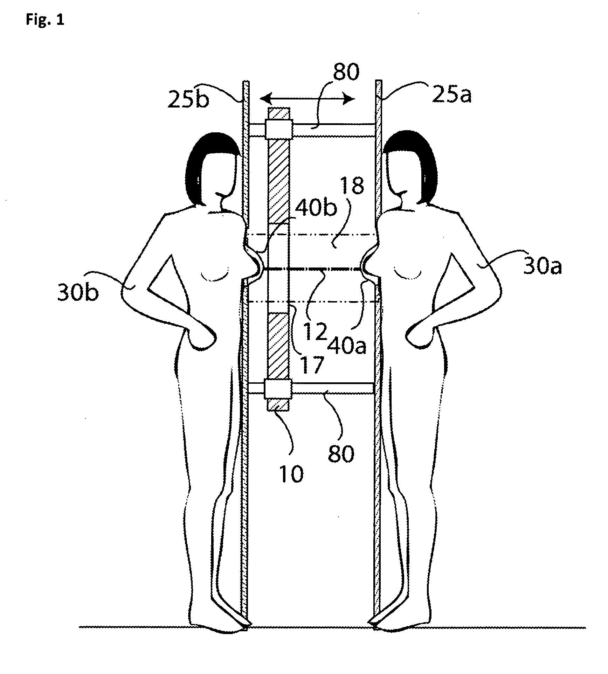

In Figur 1 ist ein erfindungsgemäßes Röntgengerät dargestellt. Die um die horizontale Drehachse 12 drehbare Gantry 10 hat eine Röntgeneinrichtung umfassend eine Röntgenröhre 15 und einen Röntgendetektor 16. Durch eine Vorschubeinrichtung 80 wird die Gantry 10 in parallel zur Drehachse 12 in Bezug auf die Fixiervorrichtung 40a, 40b verschoben. Die Verschiebung erfolgt synchron zur Drehung der Gantry 10. Die Gantry 10 ist zwischen zwei Untersuchungsräumen angeordnet. Die zu den Untersuchungsräumen angrenzenden Wände 4a, 4b verfügen jeweils über eine Brustfixierung 40a, 40b. Eine Brust der ersten Patientin 30a ist in einer Fixiervorrichtung 40a aufgenommen. Eine Brust der zweiten Patientin 30b ist in einer Fixiervorrichtung 40b aufgenommen. Vorteilhaft ist es, die Wände 25a, 25b derart zu gestalten, so dass sie die Strahlung zu den Patientinnen hin abschirmen. Mit einem Gerät können die erste Patientin 30a und die zweite Patientin 30b untersucht werden, obwohl sie sich in zwei getrennten Untersuchungsräumen befinden. Die Länge des zylinderförmigen Messfeldes 18 des Röntgengerätes wird definiert durch den maximalen Weg der Vorschubeinrichtung 80, der durch den Abstand der Trennwände 25a, 25b bestimmt ist.

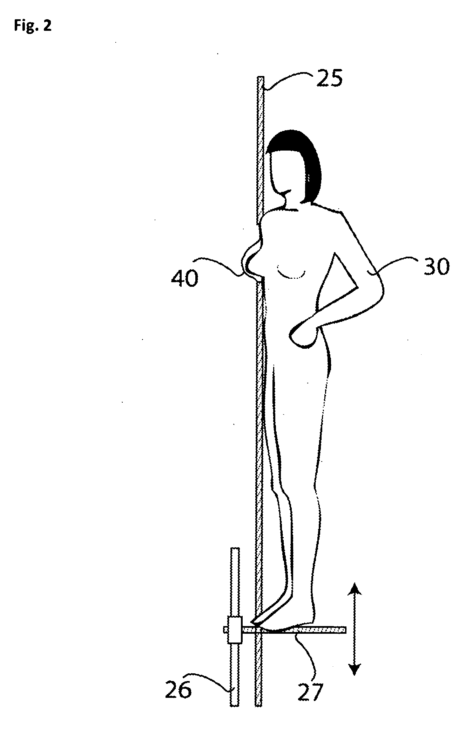

Figur 2 zeigt die Vorrichtung aus Fig. 1 mit einer Trittstufe 27, die durch einen Hubantrieb 26 so eingestellt werden kann, dass die Patientin 30 so vor der Wand 25a, 25b positioniert werden kann, dass ihre Brust bequem in der Brustfixierung 40a, 40b aufgenommen werden kann.

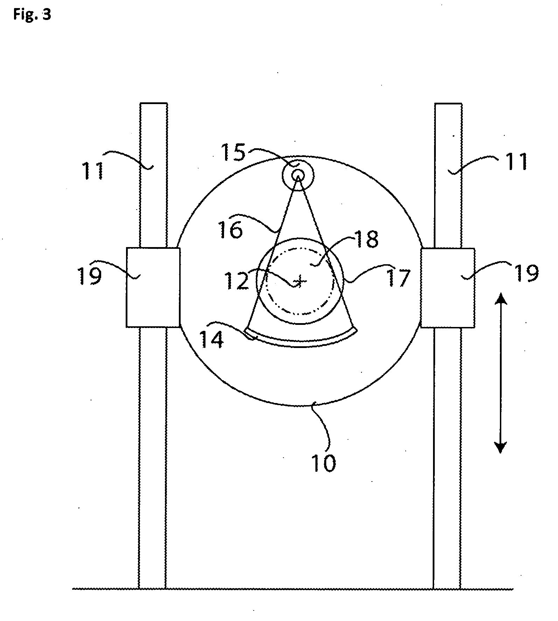

Figur 3 zeigt eine Ausgestaltung der Vorrichtung, bei der die im Wesentlichen scheibenförmige Gantry 10 zusammen mit der Brustfixierung 40 ein zusätzliches Gantryhublager 19 besitzt. Die Gantry 10 kann zusammen mit der Brustfixierung 40 über einen Gantryhubantrieb 11 in der Höhe verstellt werden. Die Gantry selbst umfasst die Röntgenröhre 15 und den Röntgendetektor 16. Der Strahlengang zwischen Röntgenröhre 15 und dem Röntgendetektor 14 ist durch den Röntgenstrahlenfächer 16 dargestellt. In diesem Beispiel begrenzt der Röntgenstrahlenfächer 16 den Durchmesser des zylinderförmigen Messfeldes 18 auf einen Wert, der kleiner als die Innenbohrung 17 der Gantry ist.

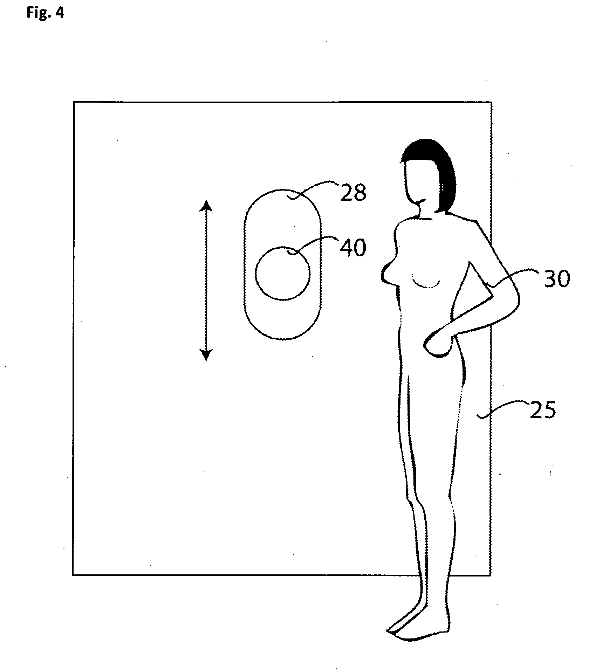

Figur 4 zeigt das Röntgengerät aus Figur 3, wobei die Wand 25a, 25b einen Wandausschnitt 28 besitzt, so dass bei jeder eingestellten Höhe die Brustfixierung 40a zugänglich ist.

### Bezugszeichenliste

- 10: Gantry
- 11: Gantryhubantrieb
- 12: Drehachse
- 14: Röntgendetektor
- 15: Röntgenröhre
- 16: Strahlenfächer
- 17: Innenbohrung der Gantry
- 18: Messfeld
- 19: Gantryhublager
- 25: Trennwand
- 26: Hubantrieb
- 27: Trittstufe
- 28: Wandausschnitt
- 30: Patient
- 40: Fixiervorrichtung
- 80: Vorschubeinrichtung

## Patentansprüche

1. Röntgengerät zur Abbildung einer Brust einer Patientin (30), mit
- einer Röntgeneinrichtung umfassend eine Röntgenröhre (15) und einen Röntgendetektor (16),
- einer Fixiervorrichtung (40a, 40b) für eine Brust (18) in einem Strahlengang zwischen der Röntgenröhre (15) und dem Röntgendetektor (16),
wobei eine um eine näherungsweise horizontale Drehachse (12) drehbare Gantry (10) mit der Röntgenröhre (15) sowie dem Röntgendetektor (16) vorgesehen ist, wobei die Gantry zur Abbildung der Brust (18) in eine kontinuierliche Rotationsbewegung versetzt werden kann, und
die Gantry (10) mit einer Vorschubeinrichtung (80) zur linearen Verschiebung der Gantry in Bezug auf die Fixiervorrichtung (40a, 40b) und in Richtung der Drehachse (12) der Gantry (10) verbunden ist, wobei die lineare Verschiebung in Abhängigkeit von der Rotationsbewegung erfolgt,
**dadurch gekennzeichnet, dass**
eine zweite Fixiervorrichtung (40b) vorgesehen ist, welche auf der der ersten Fixiervorrichtung (40a) gegenüberliegenden Seite der Gantry (10) angeordnet ist, so dass von jeder Seite der Gantry eine Brust eingeführt werden kann.

2. Röntgengerät nach Anspruch 1,
**dadurch gekennzeichnet, dass**
mindestens eine Fixiervorrichtung (40a, 40b) in eine Trennwand (25a, 25b) integriert ist.

3. Röntgengerät nach einem der vorhergehenden Ansprüche,
**dadurch gekennzeichnet, dass**
die Fixierung und/oder Stabilisierung der Brust (18) in der Fixiervorrichtung (40a, 40b) mittels Vakuum beziehungsweise Unterdruck erfolgt.

4. Röntgengerät nach einem der vorhergehenden Ansprüche,
**dadurch gekennzeichnet, dass**
die Vorrichtung wahlweise einen Standfuß zur Aufstellung auf einem Fußboden oder eine Wandhalterung zur Wandmontage hat.

5. Röntgengerät nach einem der vorhergehenden Ansprüche,
**dadurch gekennzeichnet, dass**
das Röntgengerät hinter einer Trennwand (25a, 25b) mit einem Ausschnitt für die Brustfixierung angeordnet ist.

6. Röntgengerät nach einem der vorhergehenden Ansprüche,
**dadurch gekennzeichnet, dass**
die Gantry (10) in der Höhe verstellbar und somit an verschiedene Körpergrößen der Patientin anpassbar ist.

7. Röntgengerät nach Anspruch 6,
**dadurch gekennzeichnet, dass**
eine Hubvorrichtung zur Höhenverstellung vorgesehen ist.

8. Röntgengerät nach Anspruch 7,
**dadurch gekennzeichnet, dass**
die Hubvorrichtung einen Motor aufweist.

9. Röntgengerät nach einem der vorhergehenden Ansprüche,
**dadurch gekennzeichnet, dass**
eine höhenverstellbare Trittstufe für die zu untersuchende Person vorgesehen ist.

10. Röntgengerät nach einem der vorhergehenden Ansprüche,
**dadurch gekennzeichnet, dass**
das Röntgengerät ein Spiral-CT Gerät ist.

11. Röntgengerät nach einem der vorhergehenden Ansprüche,
**dadurch gekennzeichnet, dass**
das Röntgengerät ein Sequentielles CT Gerät ist.

## Claims

1. X-Ray machine for imaging a breast of a female patient (30), comprising:
- an X-ray facility with an X-ray tube (15) and an X-ray detector (16);
- a locating device (40a, 40b) for a breast (18) in a ray path between the X- ray tube (15) and the X-ray detector (16);
in which a gantry (10) that is rotatable about an approximately horizontal rotation axis (12) is provided together with the X-ray tube (15) and also the X-ray detector (16), and in which the gantry is adapted to be set into continuous rotational motion for imaging the breast (18); and
the gantry (10) is connected with an advancing means (80) for linear displacement of the gantry relative to the locating device (40a, 40b) and along the direction of the rotation axis (12) of the gantry (10), with the linear displacement being effected in dependence upon the rotational motion;
**characterized in that**
a second locating device (40b) is provided and is disposed on the side of the gantry (10) opposite to the first locating device (40a), so that one breast can be inserted from each side of the gantry.

2. X-ray machine according to claim 1,
**characterized in that**
at least one locating device (40a, 40b) is incorporated in a separating wall (25a, 25b).

3. X-ray machine according to any one of the preceding claims,
**characterized in that**
locating or stabilizing of the breast (18) in the locating device (40a, 40b) is effected by means of vacuum or sub-pressure.

4. X-ray machine according to any one of the preceding claims,
**characterized in that**
the device optionally has a stand for setting-up on a floor, or a wall holder for wall mounting.

5. X-ray machine according to any one of the preceding claims,
**characterized in that**
the X-ray machine is disposed behind a separating wall (25a, 25b) having a cut-out portion for breast location.

6. X-ray machine according to any one of the preceding claims,
**characterized in that**
the gantry (10) is of adjustable height and thus adaptable to various body sizes of the female patient.

7. X-ray machine according to claim 6,
**characterized in that**
a lifting device is provided for adjustment of height.

8. X-ray machine according to claim 7,
**characterized in that**
the lifting device has a motor.

9. X-ray machine according to any one of the preceding claims,
**characterized in that**
a footboard of adjustable height is provided for the person to be examined.

10. X-ray machine according to any one of the preceding claims,
**characterized in that**
the X-ray machine is a spiral CT instrument.

11. X-ray machine according to any one of the preceding claims,
**characterized in that**
the X-ray machine is a sequential CT instrument.

## Revendications

1. Appareil radiographique pour l'imagerie d'un sein d'une patiente (30), avec
- une installation à rayons X comprenant un tube à rayons X (15) et un détecteur de rayons X (16),
- un dispositif de fixation (40a, 40b) pour un sein (18) dans un trajet de rayons entre le tube à rayons X (15) et le détecteur de rayons X (16),
dans lequel est prévu un bras (10) pouvant tourner autour d'un axe de rotation (12) approximativement horizontal avec le tube à rayons X (15) et le détecteur de rayons X (16), lequel bras peut être entraîné dans un mouvement de rotation continu pour l'imagerie du sein (18), et
le bras (10) est relié à un dispositif de déplacement (80) pour le déplacement linéaire du bras par rapport au dispositif de fixation (40a, 40b) et en direction de l'axe de rotation (12) du bras (10), le déplacement linéaire se produisant en fonction du mouvement de rotation, **caractérisé en ce qu'**il est prévu un deuxième dispositif de fixation (40b) qui est disposé sur le côté du bras (10) opposé au premier dispositif de fixation (40a), de sorte qu'un sein peut être introduit depuis chaque côté du bras.

2. Appareil radiographique selon la revendication 1, **caractérisé en ce qu'**au moins un dispositif de fixation (40a, 40b) est intégré dans une cloison de séparation (25a, 25b).

3. Appareil radiographique selon l'une des revendications précédentes, **caractérisé en ce que** la fixation et/ou la stabilisation du sein (18) dans le dispositif de fixation (40a, 40b) sont réalisées au moyen de vide ou d'une dépression.

4. Appareil radiographique selon l'une des revendications précédentes, **caractérisé en ce que** le dispositif possède au choix un pied d'appui pour être posé sur un sol ou une fixation murale pour un montage mural.

5. Appareil radiographique selon l'une des revendications précédentes, **caractérisé en ce que** l'appareil radiographique est disposé derrière une cloison de séparation (25a, 25b) avec une découpe pour la fixation du sein.

6. Appareil radiographique selon l'une des revendications précédentes, **caractérisé en ce que** le bras (10) est mobile en hauteur et peut ainsi être adapté à des tailles de patientes différentes.

7. Appareil radiographique selon la revendication 6, **caractérisé en ce qu'**il est prévu un dispositif de levage pour le réglage en hauteur.

8. Appareil radiographique selon la revendication 7, **caractérisé en ce que** le dispositif de levage comporte un moteur.

9. Appareil radiographique selon l'une des revendications précédentes, **caractérisé en ce qu'**il est prévu un marchepied réglable en hauteur pour la personne à examiner.

10. Appareil radiographique selon l'une des revendications précédentes, **caractérisé en ce que** l'appareil radiographique est un scanner TDM spiralé.

11. Appareil radiographique selon l'une des revendications précédentes, **caractérisé en ce que** l'appareil radiographique est un scanner TDM séquentiel.
